# EUROPEAN PATENT SPECIFICATION

(11) **EP 1 660 038 B1**
(45) Date of publication and mention of the grant of the patent: **27.08.2014**
(21) Application number: 03818189.7
(22) Date of filing: 30.07.2003
(51) Int. Cl.: A61K 9/10, A61K 31/19, A61K 31/573, A61K 47/12, A61K 47/22, A61K 8/18

(54) **STEROIDAL COMPOSITIONS CONTAINING HYDROXYCARBOXYLIC ACIDS AND METHODS OF USING THE SAME**
STEROIDALE ZUSAMMENSETZUNGEN MIT HYDROXYCARBONSÄUREN UND ANWENDUNGSVERFAHREN DAFÜR
COMPOSITIONS STEROIDIQUES CONTENANT DES ACIDES HYDROXYCARBOXYLIQUES ET PROCEDES D'UTILISATION ASSOCIES

(43) Date of publication of application: 31.05.2006
(73) Proprietor: STIEFEL LABORATORIES, INC., Coral Gables, FL 33134 (US)
(72) Inventor: Serikaku, Daniela, 331-Sâo Paulo, SP-Brazil 03334-000 (BR)
(74) Representative: Reeves, Julie Frances
(86) International application number: PCT/US2003/023723
(87) International publication number: WO 2005/016307

(56) References cited:
- WO-A1-01/85159
- WO-A2-02/096374
- US-A- 4 246 261
- US-A- 4 946 870
- US-B1- 6 479 058
- VOGEL H G ET AL: "Comparison of various pharmaceutical preparations of prednicarbate after repeated topical administration to the skin of rats.", ARZNEIMITTEL-FORSCHUNG 1985 LNKD- PUBMED:4026921, vol. 35, no. 6, 1985, pages 939-946, XP002617350, ISSN: 0004-4172
- PETRI W ET AL: "Galenische und klinisch-pharmakologische Untersuchungen zu Prednicarbate (Hoe 777)", ZEITSCHRIFT FÜR HAUTKRANKHEITEN 1986 LNKD- PUBMED:3705674, vol. 61 Suppl 1, 1986, pages 62-73, XP008131801, ISSN: 0301-0481
- Lee MG: "Lactic acid" In: Rowe RC et al. (Eds): "Handbook of Pharmaceutrical Excipients, 4th Ed.", 2003, Pharmaceutical Press, London ISBN: 0853694729 pages 319-320,

## Description

### FIELD OF THE INVENTION

The present inventive subject matter relates to pharmaceutical compositions suitable for topical administration comprising two active ingredients, lactic acid as a hydroxycarboxylic acid and prednicarbate, and sodium pyrrolidone carboxylate moisturizing agent. In a particular aspect, the two active ingredients in the present inventive compositions have a purity of at least 90% and a concentration of degradation product(s) less than about 10% of the starting concentration of the active ingredients. These compositions are used for topical medical applications, particularly to treat steroid responsive dermatoses.

### BACKGROUND OF THE INVENTION

Topical medications that include corticosteroids are known in the art as useful for treating skin conditions such as atopic dermatitis, psoriasis and other pathologies of the skin. Current steroid-containing products are available mainly as gels, lotions or ointments that are supplied in tubes or bottles and applied to an affected area of the skin by hand.

However, the results of using corticosteroids in topical treatment of, for example, psoriasis have been variable and unpredictable. In some cases topical corticosteroids seemed to improve and eradicate the psoriatic lesions, but in other cases corticosteroids appeared to be ineffective on topical administration. Drug resistance and rebound worsening are also common features when corticosteriods alone are used in the treatment of psoriasis. Accordingly, it is often required to use corticosteroids in combination with another ingredient that stabilizes and enhances the activity of the corticosteroid.

U.S. Patent No. 3,879,537 describes the use of certain α-hydroxy acids, α-keto acids, and related compounds for topical treatment of fish-scale like ichthyotic conditions in humans.

Similarly, U.S. Patent No. 3,920,835 describes the use of these certain α-hydroxy acids, α-keto acids, and their derivatives for topical treatment of dandruff, acne, and palmar and plantar hyperkeratosis. α-hydroxy acids and α-keto acids, then, were also well known in the art as having dermatological effects.

In view of these prior teachings, U.S. Patent No. 4,246,261 discloses that hydroxy acids and related compounds greatly enhance the therapeutic efficacy of corticosteroids in the topical treatment of such dermatological disorders as psoriasis, eczema, seborrheic dermatitis, and other inflammatory skin conditions.

A number of products have entered the marketplace taking advantage of this hydroxy acid-corticosteroid combination. For example, the Lacticare-HC Lotion product (Stiefel Laboratories, Inc., Coral Gables, FL) contains a combination of hydrocortisone and lactic acid. This product has been well known for use in the treatment of, for example, pruritis.

Numerous other similar products have entered the marketplace containing a combination of hydroxy acids such as lactic acid and a corticosteroid such as hydrocortisone. The majority of these products, however, contain hydrocortisone as the steroidal active ingredient.

Excessive use of hydrocortisone is well-known to exhibit a variety of undesired side effects, including blurred vision, halos around lights, an irregular heartbeat, insomnia, mood changes, weight gain, fatigue, redness, blistering, burning, itching, peeling, thinning of the skin, and stretch marks. Additionally, it is well known that children are especially sensitive to the unwanted side effects of topically administered hydrocortisone. Accordingly, there remains a need in the art for topical corticosteroid products containing a steroid other than hydrocortisone, especially for the treatment of children.

Several such topical products containing a steroid other than hydrocortisone have been sold. However, none of these teach or suggest the use of a moisturizing agent which may aid in the therapeutic effects and patient compliance with these compositions. As shown by U.S. Patent No. 5,874,974 it is desirable to include an agent having a moisturizing or emollient effect with a composition containing a steroid to supplement the curative action of the steroid and to enhance the effect of the steroid on the skin.

One deficiency of the prior art topical steroid compositions is the lack of recognition for maintaining a high purity level of the active drugs with a low amount of degradates. This deficiency is overcome with the present formulations which not only contain three essential components but also require a high drug purity and low drug degradates, which increases the effectiveness and shelf-life of the topical composition.

Accordingly, there remains a need in the art for topical steroidal compositions useful in treating a variety of dermatological disorders that contain a steroid other than hydrocortisone, a second ingredient, such as a hydroxy acid, to stabilize and enhance the activity of the steroid, and a moisturizing agent to supplement the curative activity of the steroid. There further remains a need for such topical compositions that maintain a high purity level of the active drug(s) and a low level of degradates thereof. The present inventive subject matter addresses these needs.

Topical compositions comprising prednicarbate and lactic acid are disclosed by HG Vogel and W Petri (1985) in Arzneimittel-Forschung/Drug Research 35: 939-946, and by W Petri et al. (1986) in H+G Zeitschrift für Hautkrankheiten 61: 62-73.

### SUMMARY OF THE INVENTION

The present inventive subject matter relates to a
pharmaceutical composition suitable for topical administration comprising:
1 to 10% by weight of lactic acid or a pharmaceutically acceptable salt thereof;
0.1 to 1.0% by weight of prednicarbate or a pharmaceutically acceptable salt thereof; and
1 to 8% by weight of sodium pyrrolidone carboxylate;
wherein the lactic acid and prednicarbate have a purity of at least 90% and a concentration of degradation product(s) of less than 10% of the starting concentration of said lactic acid and prednicarbate, over the shelf life of the composition; and
wherein the composition is a lotion which is an emulsion having an oil phase and an aqueous phase and said composition has a pH of 3.0 to 6.0.

In a preferred embodiment, the present inventive subject-matter relates to the above composition for use in the treatment of a steroid responsive dermatosis in a mammal by topical administration.

### DETAILED DESCRIPTION OF THE INVENTION

### Definitions

As used herein, "degradation products" refers to the product (s) produced by decomposition of one or more of the active ingredients of the present inventive compositions.

As used herein, an "extended period of time" refers to the shelf life of a composition of the present inventive subject matter, including time spent on the shelf at a pharmacy as well as the entire time period after sale of the composition during which the composition remains effective for the indicated use.

As used herein, "lactones" refers to derivatives of the subject compound(s), modified so that a hydroxyl group and a carboxylic acid group combine to form a cyclic ester, that possess the same pharmacological activity as the subject compound(s) and which are neither biologically nor otherwise undesirable. Non-limiting examples of suitable lactones include gluconolactone, galactonolactone, glucuronolactone, galacturonolactone, gulonolactone, ribonolactone, saccharic acid lactone, pantoyllactone, glucoheptonolactone, mannonolactone, and galactoheptonolactone.

As used herein, "pharmaceutically acceptable salts " refers to salts of the active compound(s) which possess the same pharmacological activity as the active compound(s) and which are neither biologically nor otherwise undesirable. A salt can be formed with, for example, organic or inorganic acids. Non-limiting examples of suitable acids include acetic acid, acetylsalicylic acid, adipic acid, alginic acid, ascorbic acid, aspartic acid, benzoic acid, benzenesulfonic acid, bisulfic acid, boric acid, butyric acid, camphoric acid, camphorsulfonic acid, carbonic acid, citric acid, cyclopentanepropionic acid, digluconic acid, dodecylsulfic acid, ethanesulfonic acid, formic acid, fumaric acid, glyceric acid, glycerophosphoric acid, glycine, glucoheptanoic acid, gluconic acid, glutamic acid, glutaric acid, glycolic acid, hemisulfic acid, heptanoic acid, hexanoic acid, hippuric acid, hydrobromic acid, hydrochloric acid, hydroiodic acid, hydroxyethanesulfonic acid, lactic acid, maleic acid, malic acid, malonic acid, mandelic acid, methanesulfonic acid, mucic acid, naphthylanesulfonic acid, naphthylic acid, nicotinic acid, nitrous acid, oxalic acid, pelargonic, phosphoric acid, propionic acid, saccharin, salicylic acid, sorbic acid, succinic acid, sulfuric acid, tartaric acid, thiocyanic acid, thioglycolic acid, thiosulfuric acid, tosylic acid, undecylenic acid, ethanolamine, naturally and synthetically derived amino acids. Non-limiting examples of base salts include ammonium salts; alkali metal salts, such as sodium and potassium salts; alkaline earth metal salts, such as calcium and magnesium salts; salts with organic bases, such as dicyclohexylamine salts; methyl-D-glucamine; and salts with amino acids, such as arginine, lysine, and so forth. Also, the basic nitrogen-containing groups can be quaternized with such agents as lower alkyl halides, such as methyl, ethyl, propyl, and butyl chlorides, bromides, and iodides; dialkyl sulfates, such as dimethyl, diethyl, dibutyl, and diamyl sulfates; long chain halides, such as decyl, lauryl, myristyl, and stearyl chlorides, bromides, and iodides; asthma halides, such as benzyl and phenethyl bromides; and others. Water or oil-soluble or dispersible products are thereby obtained.

Other terms as used herein are meant to be defined by their well-known meanings in the art.

### Topical Pharmaceutical Compositions

The present inventive pharmaceutical compositions suitable for topical administration contain two active ingredients: an α-hydroxy acid , ie. lactic acid, and prednicarbate. The presence of these two different active ingredients conveys a synergistic, or a greater than additive, effect upon application of the present inventive compositions to the skin. That is, the present inventive compositions containing both of these active ingredients produce a greater medical effect than would be exhibited by adding the medical effects of an α-hydroxy acid and prednicarbate applied to the same skin separately.

Additionally, the present inventive pharmaceutical compositions contain a moisturizing agent, i.e. sodium pyrrolidone carboxylate moisturizing agent. This moisturizing agent acts to further enhance the effects and curative action of the prednicarbate on the skin. Further, the moisturizing agent moisturizes the skin, avoiding normal side effects of corticosteroid use such as drying, redness, blistering, burning, itching, and peeling of the skin. Accordingly, the addition of the moisturizing agent to the present compositions will improve patient compliance with a prescribed treatment regimen.

The present inventive compositions are formed as an oil-in-water emulsion, i.e. an emulsion having an oil phase and an aqueous phase. Preferably, the oil phase of the emulsion comprises an oily material and an emulsifier to aid in formation of the emulsion. More preferably, the oil phase contains at least two emulsifiers.

The first active ingredient, lactic acid or pharmaceutically acceptable salt thereof, is preferably contained in the aqueous phase of the emulsion. The aqueous phase additionally preferably contains the moisturizing agent of the present inventive compositions, i.e. the pyrrolidone carboxylate salt. The pH of this aqueous phase, if required, is adjusted to a range of about 3.0 to about 6.0 before it is combined with the oil phase to form the emulsion. In a particularly preferred embodiment, the pH of the aqueous phase is adjusted to a pH range of about 4.0 to about 5.0.

Once the oil phase and the aqueous phase are combined to form the emulsion, the second active ingredient, prednicarbate or a pharmaceutically acceptable salt thereof, is solubilized in a suitable solvent and dispersed throughout the emulsion.

Since the emulsion is an oil-in-water emulsion having water as the major component, the final composition will have a pH mirroring that of the aqueous phase. Accordingly, the pH of the final composition ranges from 3.0 to 6.0. In a particularly preferred embodiment, the pH of the final composition ranges from about 4.0 to about 5.0.

These particular emulsion and pH characteristics convey to the present compositions the unique advantages of being able to maintain a high purity level and a low concentration of degradation products of the active ingredients. The high purity level and low concentration of degradation products permits the present inventive compositions to have a longer shelf life and increased pharmaceutical effectiveness when compared with other corticosteroid products previously known in the art.

In this regard, the present inventive compositions maintain a purity level of at least 90%, preferably at least 92.5%, more preferably at least 95% of each of the active ingredients over an extended period of time.

Likewise, the present inventive compositions are able to maintain a low concentration of degradation product(s) of the active ingredients over an extended period of time. In this regard, the present compositions will maintain a concentration of degradation product(s) less than about 10%, preferably less than about 7.5%, more preferably less than about 5% of the starting concentration of each of the active ingredients. These advantageous properties were previously unknown in the prior art compositions.

### α-Hydroxy Acids

The present inventive compositions contain 1 to 10% by weight of lactic acid as an α-hydroxy acid or a pharmaceutically acceptable salt thereof as a first active ingredient.

In a preferred embodiment, the present inventive compositions contain about 3 to about 7% by weight of the α-hydroxy acid or a pharmaceutically acceptable salt thereof.

It is an essential aspect for the present inventive compositions to maintain a purity level of at least 90%, preferably at least 92.5%, more preferably at least 95% of the α-hydroxy acid over an extended period of time. Likewise, the present inventive compositions are able to maintain a low concentration of degradation product(s) of the α-hydroxy acid, namely less than about 10%, preferably less than about 7.5%, more preferably less than about 5% of the starting concentration of the α-hydroxy acid over an extended period of time.

The α-hydroxy acid is lactic acid or a salt thereof.

### Prednicarbate

The present inventive compositions further contain 0.1 to 1.0% by weight of the prednicarbate or a pharmaceutically acceptable salt thereof. In a most preferred embodiment, the present inventive compositions contain about 0.15 to about 0.5% by weight of the prednicarbate or a pharmaceutically acceptable salt thereof.

It is an essential aspect for the present inventive compositions to maintain a purity level of at least 90%, preferably at least 92.5%, more preferably at least 95% of the prednicarbate over an extended period of time. Likewise, the present inventive compositions are able to maintain a low concentration of degradation product(s) of the prednicarbate, e.g. less than about 10%, preferably less than about 7.5%, more preferably less than about 5% of the starting concentration of the prednicarbate, over an extended period of time.

Prednicarbate is especially useful in the present inventive compositions since children and those having sensitive skin more easily tolerate it than other known steroids, such as hydrocortisone. Accordingly, by virtue of the presence of prednicarbate rather than hydrocortisone, the present inventive compositions permit a greater frequency of administration and a greater amount of drug to be delivered to children and those with sensitive skin.

### Moisturizing Agents

The present inventive compositions further contain as an essential component a moisturizing agent, 1 to 8% by weight of sodium pyrrolidone carboxylate . In a most preferred embodiment, the present inventive compositions contain about 3 to about 7% by weight of the pyrrolidone carboxylate salt.

The addition of the moisturizing agent to the present inventive pharmaceutical compositions enhances the effects and curative action of the prednicarbate on the skin. Further, the moisturizing agent moisturizes the skin, avoiding normal side effects of corticosteroid use such as drying, redness, blistering, burning, itching, and peeling of the skin. Accordingly, the addition of the moisturizing agent to the present compositions will improve patient compliance with a prescribed treatment regimen.

### Additional Ingredients

The present inventive compositions are formed as an oil-in-water emulsion having an oil phase and an aqueous phase. In this regard, the oil phase of the emulsion comprises an oily material and at least one emulsifier to aid in formation of the emulsion.

Non-limiting exemplary oily materials include mineral oil, petrolatum, petroleum derivatives, fatty acids, fatty acid derivatives, fatty alcohols, fatty alcohol derivatives, paraffins, and mixtures thereof.

At least one emulsifier is used in the present inventive compositions to form the emulsion. In a preferred embodiment, at least two emulsifiers are present in the oil phase to help form the emulsion. Preferred, non-limiting examples of emulsifiers used in the present inventive compositions include polyoxyethylene sorbitan fatty acid esters, sorbitan fatty acid esters, propylene glycol stearate, glyceryl monostearate, polyethylene glycol, fatty alcohols, polymeric ethylene oxide-propylene oxide block polymers (Pluronics), derivatives thereof, pharmaceutically acceptable salts thereof, and mixtures thereof. In a preferred embodiment, the emulsifiers used in the present inventive compositions are either naturally or synthetically prepared.

Particularly preferred emulsifies useful in the present inventive compositions include but are not limited to stearyl alcohol and polyoxyethylene(20) cetostearyl ether (Ceteareth-20), and glyceryl stearate and polyethyleneglycol-100 (PEG-100)/glyceryl stearate.

The aqueous phase forms the major portion of the present emulsion compositions. Accordingly, the present compositions preferably comprise about 50 to about 98% by weight water. In a particularly preferred embodiment, the present compositions comprise about 55 to about 85% by weight water.

The present inventive compositions may further comprise several additional excipients commonly known to those of ordinary skill in the art as useful in topical compositions. Several non-limiting examples of such additional excipients include antioxidants, chelates, preservatives, emollients, humectants, fluid alkyl alcohols, thickening agents, pH modifier, and mixtures thereof.

Non-limiting examples of specific antioxidants useful in the present inventive compositions include ascorbic acid, fumaric acid, malic acid, alpha tocopherol, ascorbic acid palmitate, butylated hydroxyanisole, propyl gallate, sodium ascorbate, sodium metabisulfite, and mixtures thereof.

Non-limiting examples of specific preservatives useful in the present inventive compositions include methylparaben, benzalkonium chloride, propylparaben, benzoic acid, EDTA, phenolic acid, sorbic acid, benzyl alcohol, isopropyl alcohol, benzethonium chloride, bronopol, butylparaben, cetrimide, chlorhexidine, chlorobutanol, chlorocresol, cresol, ethylparaben, glycerol, imidurea, phenol, phenoxyethanol, phenylethyl alcohol, potassium sorbate, propylene glycol, sodium benzoate, sodium propionate, sorbic acid, thimerosol, and mixtures thereof. A particularly preferred preservative in this regard is methylparaben.

Non-limiting examples of specific emollients useful in the present inventive compositions include myristyl lactate, isopropyl palmitate, light liquid paraffin, cetearyl alcohol, lanolin, mineral oil, petrolatum, ceryl esters wax, cholesterol, glycerol, glycerol monostearate, isopropyl myristate, lecithin, and mixtures thereof. Particularly preferred emollients in this regard are myristyl lactate, isopropyl palmitate, and light liquid paraffin.

Non-limiting examples of specific humectants useful in the present inventive compositions include glycerin, propylene glycol, sorbitol, and triacetin.

Non-limiting examples of specific fluid alkyl alcohols useful in the present inventive compositions include ethanol, isopropyl alcohol, octodecyl alcohol, propyl alcohol, butanol, and pentanol. A particularly preferred fluid alkyl alcohol in this regard is ethanol. Non-limiting examples of specific thickening agents useful in the present inventive compositions include cetyl alcohol, Carbomers, acrylates/C10-30 alkyl acrylate crosspolymers, hydroxyethylcellulose, hydroxypropylcellulose, polyethylene oxide, and mixtures thereof. Particularly preferred thickening agents in this regard are cetyl alcohol, Carbomer 940, and acrylates/C10-30 alkyl acrylate crosspolymer.

The pH modifiers useful in the present inventive compositions include acids, bases, and mixtures thereof. Preferred non-limiting examples of pH modifiers in this regard include acetic acid, acetylsalicyclic acid, ascorbic acid, boric acid, carbonic acid, citric acid, formic acid, ethanesulfonic acid, fumaric acid, glycerophosphoric acid, hippuric acid, hydrochloric acid, maleic acid, methanesulfonic acid, nitrous acid, oxalic acid, phosphoric acid, saccharin, sorbic acid, sulfuric acid, thiosulfuric acid, undecylenic acid, ethanolamine, triethanolamine, sodium carbonate, sodium acetate, sodium hydrogen phosphate, sodium dihydrogen phosphate, sodium citrate, sodium bicarbonate, sodium hydroxide, and mixtures thereof.

In a particularly preferred embodiment, the pH modifier contains a hydroxyl group. A most preferred pH modifier containing a hydroxyl group useful in the present inventive compositions is sodium hydroxide.

### Methods of Treatment

Several specific steroid responsive dermatoses may be treated according to the present inventive methods. Examplary among these dermatoses are contact dermatitis, eczema, atopic dermatitis, ichthyosis, psoriasis, xeroderma, seborrheic dermatitis, nummular dermatitis, stasis dermatitis, lichen simplex chronicus, dermatophytids, candidiasis, scabies, pityriasis rosea, lichen planus, pityriasis rubra pilaris, bullous pemphigoid, miliaria, acute and chronic eczema, lupus erythematosis, photoallergic reactions, pruritis, and combinations thereof. Other steroid responsive dermatoses known to those of ordinary skill in the art are further contemplated as within the scope of the present inventive subject matter.

The steroid responsive dermatosis treated according to the present inventive methods can have a variety of causes. Several non-limiting examples of such causes include hypersensitivity, IgE mediation, anti-membrane antibody, immune complex disease, cell mediated immunity, and combinations thereof.

The steroid responsive dermatosis may also be caused by an insult to a tissue of the mammal having the dermatosis. Several non-limiting examples of such insults include a physical insult, a chemical insult, an environmental insult, a topically mediated insult, an internally mediated insult, and combinations thereof.

Additionally, said steroid responsive dermatosis may be a secondary physiologic response to a primary disease. Several non-limiting examples of such primary diseases causative of the steroid responsive dermatosis include an infection, an allergic response, a hyperproliferative disorder, an immunologic disorder, a metabolic disorder, a drug induced response, a disorder related to proper or improper organ function, and combinations thereof.

The steroid responsive dermatosis may cause a variety of symptoms in the mammal afflicted therewith. Non-limiting examples of possible symptoms include inflammation, redness, tissue disruption, tissue deformation, exudates, crusting, pain, pruritis, and mixtures thereof.

In addition to treating steroid responsive dermatosis, the present inventive methods also contemplate using the inventive compositions described herein for treating diseases tissue in a mammal.

### Methods of Production

The present inventive subject matter further relates to a process for preparing a pharmaceutical composition suitable for topical administration comprising an emulsion, said process comprising:
1) preparing an oil phase comprising an oily material selected from the group consisting of mineral oil, petrolatum, petroleum derivatives, fatty acids, fatty acid derivatives, fatty alcohols, fatty alcohol derivatives, paraffins, and mixtures thereof and at least two emulsifiers;
2) preparing an aqueous phase comprising the α-hydroxy acid or a pharmaceutically acceptable salt thereof having a purity of at least 90% and a concentration of degradation product(s) less than about 10% of the starting concentration of said α-hydroxy acid and the pyrrolidone carboxylate salt;
3) adjusting the pH of said aqueous phase to a range of 3.0 to 6.0.
4) adding said oil phase to said aqueous phase while mixing at a temperature of about 55 to about 85 °C to obtain a homogenous emulsion;
5) cooling said emulsion to a temperature of about 25 to about 45 °C;
6) solubilizing prednicarbate or a pharmaceutically acceptable salt thereof having a purity of at least 90% and a concentration of degradation product (s) less than about 10% of the starting concentration of said prednicarbate in a lower alkyl alcohol and dispersing said prednicarbate throughout said emulsion; and
7) recovering a topical emulsion pharmaceutical composition.

In a preferred embodiment of the present inventive subject matter, the oil phase is prepared by mixing an oily material and at least two emulsifiers at a temperature of about 55 to about 85 °C. In a particularly preferred embodiment, the oil phase is prepared by further mixing a thickening agent, an emollient, and a preservative with the oily material and the at least two emulsifiers.

In another preferred embodiment of the present inventive subject matter, the aqueous phase is prepared by first mixing a preservative followed by a polymer thickening agent in purified water at a temperature of about 55 to about 85 °C before adding the α-hydroxy acid or a pharmaceutically acceptable salt thereof and a pyrrolidone carboxylate salt. Once these ingredients are mixed, a pH modifier is added to the aqueous phase to ensure a pH of 3.0 to 6.0, preferably about 4.0 to about 5.0. In a particularly preferred embodiment, the pH is adjusted by adding sodium hydroxide to the aqueous phase. This pH represents the final pH of the composition. It is necessary to adjust the pH of the aqueous phase before addition of the oil phase to ensure that the oil phase does not intermingle with the aqueous phase, destroying the emulsion, during addition of the pH modifier.

In a particularly key aspect of the present inventive process, the emulsion is cooled from a temperature of about 55 to about 85 °C to a temperature of about 25 to about 45 °C before the prednicarbate is dispersed therein. This cooling step is particularly important because a substantial amount of prednicarbate degradation products will form at temperatures above 63 °C. Accordingly, it is necessary to cool the emulsion before adding the prednicarbate to maintain the high prednicarbate purity and low amount of prednicarbate degradation products essential to the present inventive compositions.

Further contemplated as within the scope of the present inventive subject matter are pharmaceutical compositions produced according to the above-described process, wherein the α-hydroxy acid and the prednicarbate in said compositions each maintain a purity of at least 90% and a concentration of degradation product(s) less than about 10% of the starting concentration of the α-hydroxy acid and the prednicarbate. If produced according to the present inventive process, these compositions exhibit chemical and physical stability suitable for topical administration.

The compositions produced according to these processes are used in a lotion.

These compositions can be placed in a suitable containment vessel comprising a product contact surface composed of a material selected from the group consisting of glass, plastic, steel, stainless steel, aluminum, Teflon, polymeric structure, ceramic structure, alloys, and mixtures thereof. These containment vessels are used to facilitate manufacturing, handling, processing, packaging, storage, and administration of said composition.

### Dosage

Appropriate dosage levels for the active agents contemplated in the present inventive subject matter are well known to those of ordinary skill in the art. Dosage levels on the order of about 0.001 mg to about 5,000 mg per kilogram body weight of the active therapeutic compounds or compositions are known to be useful in the treatment of the diseases, disorders, and conditions contemplated in the present invention. Typically, this effective amount of the active therapeutic agents will generally comprise from about 0.1 mg to about 100 mg per kilogram of patient body weight per day. Moreover, it will be understood that this dosage of active therapeutic agents can be administered in a single or multiple dosage units to provide the desired therapeutic effect. If desired, other therapeutic agents can be employed in conjunction with those provided by the present inventive subject matter.

As previously discussed, excessive use of hydrocortisone is well-known to exhibit a variety of undesired side effects, including redness, blistering, peeling, thinning of the skin, and stretch marks. These hydrocortisone side effects are especially pronounced in children and those having sensitive skin. The present inventive compositions solve these art-recognized problems since they contain the steroid prednicarbate, which is more easily tolerated by children and those having sensitive skin, rather than hydrocortisone. Accordingly, the present inventive compositions are especially formulated for pediatric use and for administration to sensitive skin. The present inventive compositions permit a greater frequency of administration and a greater amount of drug to be delivered to children and those with sensitive skin due to inclusion of the steroid prednicarbate rather than hydrocortisone.

The present inventive compositions may be given in a single or multiple doses daily. In a preferred embodiment, the present inventive compositions are given from one to three times daily. Starting with a low dose twice daily and slowly working up to higher doses if needed is a preferred strategy. The amount of active ingredients that may be combined with the carrier materials to produce a single dosage form will vary depending upon the host treated, the nature of the disease, disorder, or condition, and the nature of the active ingredients.

It is understood, however, that a specific dose level for any particular patient will depend upon a variety of factors well known in the art, including the activity of the specific compound employed; the age, body weight, general health, sex and diet of the patient; the time of administration; the rate of excretion; drug combination; the severity of the particular disorder being treated; and the form of administration. One of ordinary skill in the art would appreciate the variability of such factors and would be able to establish specific dose levels using no more than routine experimentation.

The optimal pharmaceutical formulations will be determined by one skilled in the art depending upon considerations such as the particular drug or drug combination and the desired dosage. See, for example, "Remington's Pharmaceutical Sciences", 18th ed. (1990, Mack Publishing Co., Easton, PA 18042), pp. 1435-1712.

Such formulations may influence the physical state, stability, rate of *in vivo* release, and rate of *in vivo* clearance of the therapeutic agents.

### EXAMPLES

The following examples are illustrative of the present inventive subject matter and are not intended to be limitations thereon. All polymer molecular weights are mean average molecular weights. All percentages are based on the percent by weight of the final delivery system or formulation prepared unless otherwise indicated and all totals equal 100% by weight.

### EXAMPLE 1

The following example illustrates the preparation of a lotion of the present inventive subject matter:

| | % W/W |
|---|---|
| Purified Water | 64.467 |
| Cetyl Alcohol | 0.590 |
| Stearyl Alcohol (and) Ceteareth-20 | 2.160 |
| Carbomer 940 | 0.600 |
| Glyceryl Stearate PEG-100/ Glyceryl Stearate | 1.4200 |
| Sodium Hydroxide | 2.000 |
| Myristyl Lactate | 0.800 |
| Methyl paraben | 0.050 |
| Isopropyl Palmitate | 3.920 |
| Sodium pyrrolidone carboxylate | 5.200 |
| Propylparaben | 0.150 |
| Light liquid paraffin | 9.520 |
| Lactic acid | 5.573 |
| Anhydrous ethanol | 3.000 |
| Acrylates/C10-30 Alkyl Acrylate Crosspolymer | 0.300 |
| Prednicarbate | 0.250 |
| | 100.0% |

### Preparation of the composition:

1. Combine the materials cetyl alcohol, myristyl lactate, light liquid paraffin, isopropyl palmitate, propylparaben, stearyl alcohol (and) Ceteareth-20, and Glyceryl Stearate PEG-100/Glyceryl Stearate, mix and heat to 70 °C ± 1 °C to form an oil phase.
2. Heat purified water to 70 °C ± 1 °C, add methylparaben and mix until clear. Add Carbomer 940 and Acrylates/C10-30 Alkyl Acrylate Crosspolymer and mix. Add sodium pyrrolidone carboxylate and lactic acid and mix to form an aqueous phase. In a separate vessel dissolve sodium hydroxide in water and add to the aqueous phase.
3. Add the oil phase to the aqueous phase and mix, maintaining the temperature of 70 °C ± 1 °C to form an emulsion. Cool to 35 °C with mixing. Solubilize prednicarbate in anhydrous ethanol, add to the emulsion, and mix.

### EXAMPLE 2

A patient is suffering from contact dermatitis. A pharmaceutical composition of the present inventive subject matter is topically administered to the patient. It would be expected that the patient would improve his/her condition or recover.

### EXAMPLE 3

A patient is suffering from eczema. A pharmaceutical composition of the present inventive subject matter is topically administered to the patient. It would be expected that the patient would improve his/her condition or recover.

### EXAMPLE 4

A patient is suffering from atopic dermatitis. A pharmaceutical composition of the present inventive subject matter is topically administered to the patient. It would be expected that the patient would improve his/her condition or recover.

### EXAMPLE 5

A patient is suffering from ichthyosis. A pharmaceutical composition of the present inventive subject matter is topically administered to the patient. It would be expected that the patient would improve his/her condition or recover.

### EXAMPLE 6

A patient is suffering from psoriasis. A pharmaceutical composition of the present inventive subject matter is topically administered to the patient. It would be expected that the patient would improve his/her condition or recover.

### EXAMPLE 7

A patient is suffering from xeroderma. A pharmaceutical composition of the present inventive subject matter is topically administered to the patient. It would be expected that the patient would improve his/her condition or recover.

### EXAMPLE 8

A patient is suffering from seborrheic dermatitis. A pharmaceutical composition of the present inventive subject matter is topically administered to the patient. It would be expected that the patient would improve his/her condition or recover.

### EXAMPLE 9

A patient is suffering from nummular dermatitis. A pharmaceutical composition of the present inventive subject matter is topically administered to the patient. It would be expected that the patient would improve his/her condition or recover.

### EXAMPLE 10

A patient is suffering from stasis dermatitis. A pharmaceutical composition of the present inventive subject matter is topically administered to the patient. It would be expected that the patient would improve his/her condition or recover.

### EXAMPLE 11

A patient is suffering from lichen simplex chronicus. A pharmaceutical composition of the present inventive subject matter is topically administered to the patient. It would be expected that the patient would improve his/her condition or recover.

### EXAMPLE 12

A patient is suffering from dermatophytids. A pharmaceutical composition of the present inventive subject matter is topically administered to the patient. It would be expected that the patient would improve his/her condition or recover.

### EXAMPLE 13

A patient is suffering from candidiasis. A pharmaceutical composition of the present inventive subject matter is topically administered to the patient. It would be expected that the patient would improve his/her condition or recover.

### EXAMPLE 14

A patient is suffering from scabies. A pharmaceutical composition of the present inventive subject matter is topically administered to the patient. It would be expected that the patient would improve his/her condition or recover.

### EXAMPLE 15

A patient is suffering from pityriasis rosea. A pharmaceutical composition of the present inventive subject matter is topically administered to the patient. It would be expected that the patient would improve his/her condition or recover.

### EXAMPLE 16

A patient is suffering from lichen planus. A pharmaceutical composition of the present inventive subject matter is topically administered to the patient. It would be expected that the patient would improve his/her condition or recover.

### EXAMPLE 17

A patient is suffering from pityriasis rubra pilaris. A pharmaceutical composition of the present inventive subject matter is topically administered to the patient. It would be expected that the patient would improve his/her condition or recover.

### EXAMPLE 18

A patient is suffering from bullous pemphigoid. A pharmaceutical composition of the present inventive subject matter is topically administered to the patient. It would be expected that the patient would improve his/her condition or recover.

### EXAMPLE 19

A patient is suffering from miliaria. A pharmaceutical composition of the present inventive subject matter is topically administered to the patient. It would be expected that the patient would improve his/her condition or recover.

### EXAMPLE 20

A patient is suffering from acute eczema. A pharmaceutical composition of the present inventive subject matter is topically administered to the patient. It would be expected that the patient would improve his/her condition or recover.

### EXAMPLE 21

A patient is suffering from chronic eczema. A pharmaceutical composition of the present inventive subject matter is topically administered to the patient. It would be expected that the patient would improve his/her condition or recover.

### EXAMPLE 22

A patient is suffering from lupus erythematosis. A pharmaceutical composition of the present inventive subject matter is topically administered to the patient. It would be expected that the patient would improve his/her condition or recover.

### EXAMPLE 23

A patient is suffering from photoallergic reactions. A pharmaceutical composition of the present inventive subject matter is topically administered to the patient. It would be expected that the patient would improve his/her condition or recover.

### EXAMPLE 24

A patient is suffering from pruritis. A pharmaceutical composition of the present inventive subject matter is topically administered to the patient. It would be expected that the patient would improve his/her condition or recover.

## Claims

1. A pharmaceutical composition suitable for topical administration comprising:
1 to 10% by weight of lactic acid or a pharmaceutically acceptable salt thereof;
0.1 to 1.0% by weight of prednicarbate or a pharmaceutically acceptable salt thereof; and
1 to 8% by weight of sodium pyrrolidone carboxylate;
wherein the lactic acid and prednicarbate have a purity of at least 90% and a concentration of degradation product(s) of less than 10% of the starting concentration of said lactic acid and prednicarbate, over the shelf life of the composition; and
wherein the composition is a lotion which is an emulsion having an oil phase and an aqueous phase and said composition has a pH of 3.0 to 6.0.

2. The pharmaceutical composition of claim 1, comprising 3 to 7% by weight of said lactic acid, 0.15 to 0.5% by weight of said prednicarbate, and 3 to 7% by weight of said sodium pyrrolidone carboxylate.

3. The pharmaceutical composition of claim 1 or 2, wherein said composition has a pH of 4.0 to 5.0.

4. The pharmaceutical composition of any one of claims 1 to 3, wherein said oil phase comprises an oily material selected from mineral oil, petrolatum, petroleum derivatives, fatty acids, fatty acid derivatives, fatty alcohols, fatty alcohol derivatives, paraffins, and mixtures thereof.

5. The pharmaceutical composition of any one of claims 1 to 4, wherein said emulsion is formed using an emulsifier selected from polyoxyethylene sorbitan fatty acid esters, sorbitan fatty acid esters, propylene glycol stearate, glyceryl monostearate, polyethylene glycol, fatty alcohols, polymeric ethylene oxide-propylene oxide block polymers, derivatives thereof, pharmaceutically acceptable salts thereof, and mixtures thereof.

6. The pharmaceutical composition of claim 5, wherein said emulsifier is a combination of stearyl alcohol and polyoxyethylene(20) cetostearyl ether, and glyceryl stearate and polyethyleneglycol-100/glyceryl stearate.

7. The pharmaceutical composition of any one of claims 1 to 6, wherein said lactic acid and said sodium pyrrolidone carboxylate are present in said aqueous phase.

8. The pharmaceutical composition of any one of claims 1 to 7, wherein said prednicarbate is dispersed in the emulsion.

9. The pharmaceutical composition of any one of claims 1 to 8, wherein said composition comprises 50 to 98% by weight of water.

10. The pharmaceutical composition of any one of claims 1 to 9, wherein the composition further comprises an excipient selected from antioxidants, chelates, preservatives, emollients, humectants, fluid alkyl alcohols, thickening agents, pH modifiers, and mixtures thereof.

11. The pharmaceutical composition of claim 10, wherein said composition comprises a pH modifier.

12. The pharmaceutical composition of claim 11, wherein said pH modifier is sodium hydroxide.

13. The composition according to any one of claims 1 to 12 for use in the treatment of a steroid responsive dermatosis in a mammal by topical administration.

14. The composition for use according to claim 13, wherein said steroid responsive dermatosis is selected from contact dermatitis, eczema, atopic dermatitis, ichthyosis, psoriasis, xeroderma, seborrheic dermatitis, nummular dermatitis, stasis dermatisis, lichen simplex chronicus, dermatophytids, candidiasis, scabies, pityriasis rosea, lichen planus, pityriasis rubra pilaris, bullous pemphigoid, miliaria, acute and chronic eczema, lupus erythematosis, photoallergic reactions, pruritis, and combinations thereof.

## Patentansprüche

1. Eine pharmazeutische Zusammensetzung, geeignet zur topischen Verabreichung, umfassend:
1 bis 10 Gew.-% Milchsäure oder ein pharmazeutisch verträgliches Salz davon;
0,1 bis 1,0 Gew.-% Prednicarbat oder ein pharmazeutisch verträgliches Salz davon; und
1 bis 8 Gew.-% Natriumpyralidoncarboxylat;
wobei die Milchsäure und das Prednicarbat eine Reinheit von mindestens 90 % und eine Konzentration von Abbauprodukt(en) von weniger als 10 % der Anfangskonzentration der Milchsäure und des Prednicarbats über die Lagerbeständigkeit der Zusammensetzung aufweisen; und
wobei die Zusammensetzung eine Lotion ist, welche eine Emulsion mit einer Ölphase und einer wässrigen Phase ist, und die Zusammensetzung einen pH von 3,0 bis 6,0 hat.

2. Die pharmazeutische Zusammensetzung nach Anspruch 1, umfassend 3 bis 7 Gew.-% der Milchsäure, 0,15 bis 0,5 Gew.-% des Prednicarbats und 3 bis 7 Gew.-% des Natriumpyrrolidoncarboxylats.

3. Die pharmazeutische Zusammensetzung nach Anspruch 1 oder 2, wobei die Zusammensetzung einen pH von 4,0 bis 5,0 hat.

4. Die pharmazeutische Zusammensetzung nach einem der Ansprüche 1 bis 3, wobei die Ölphase ein öliges Material, ausgewählt aus Mineralöl, Petrolatum, Petroleumderivaten, Fettsäuren, Fettsäurederivaten, Fettalkoholen, Fettalkoholderivaten, Paraffinen und Gemischen davon, umfasst.

5. Die pharmazeutische Zusammensetzung nach einem der Ansprüche 1 bis 4, wobei die Emulsion unter Verwendung eines Emulgators, ausgewählt aus Polyoxyethylensorbitanfettsäureestern, Sorbitanfettsäureestern, Propylenglyeolstearat, Glycerylmonostearat, Palyethytenglykol, Fettalkoholen, polymeren Ethylenoxid-Propylenoxid-Blockpolymeren, Derivaten davon, pharmazeutisch verträglichen Salzen davon und Gemischen davon, gebildet wird.

6. Die pharmazeutische Zusammensetzung nach Anspruch 5, wobei der Emulgator eine Kombination von Stearylakohol und Polyoxyethylen-(20)-cetostearylether, und Glycerylstearat und Polyethylenglykol-100/Glycerolstearat ist.

7. Die pharmazeutische Zusammensetzung nach einem der Ansprüche 1 bis 6, wobei die Milchsäure und das Natriumpyrrolidoncarboxylat in der wässrigen Phase vorliegen.

8. Die pharmazeutische Zusammensetzung nach einem der Ansprüche 1 bis 7, wobei das Prednicarbat in der Emulsion dispergiert ist.

9. Die pharmazeutische Zusammensetzung nach einem der Ansprüche 1 bis 8, wobei die Zusammensetzung 50 bis 98 Gew-% Wasser umfasst.

10. Die pharmazeutische Zusammensetzung nach einem der Ansprüche 1 bis 9, wobei die Zusammensetzung ferner einen Hilfsstoff, ausgewählt aus Antioxidantien, Chelaten, Konservierungsmitteln, Weichmachern, Befeuchtungsmitteln, Fluid-Alkylalkoholen, Verdickungsmitteln, pH-Modifizierungsmitteln und Gemischen davon, umfasst.

11. Die pharmazeutische Zusammensetzung nach Anspruch 10, wobei die Zusammensetzung ein pH-Modifizierungsmittel umfasst.

12. Die pharmazeutische Zusammensetzung nach Anspruch 11, wobei das pH-Modifizierungsmittel Natriumhydroxid ist.

13. Die Zusammensetzung nach einem der Ansprüche 1 bis 12, zur Verwendung bei der Behandlung einer auf Steroid ansprechenden Dermatose in einem Säugetier durch topische Verabreichung.

14. Die Zusammensetzung zur Verwendung nach Anspruch 13, wobei die auf Steroid ansprechende Dermatose aus Kontaktdermatitis, Ekzem, atopsicher Dermatitis, Ichthyosis, Psoriasis, Xerodermie, seborrheischem Ekzem, nummulärem Ekzem, Stauungsekzem, Lichen simplex chronicus, Dermatophyten, Kandidose, Scabies, Pityriasis rosea, Lichen planus, Pityriasis rubra pilaris, bullösem Pemphigoid, Miliaria, akuten und chronischen Ekzemen, Lupus erythematosis, photoallergischen Reaktionen, Pruritis und Kombinationen davon, ausgewählt ist.

## Revendications

1. Composition pharmaceutique convenant à l'administration topique, comprenant :
1 à 10 % en poids d'acide lactique ou d'un de ses sels pharmaceutiquement acceptables ;
0, 1 à 1,0 % en poids de prednicarbate ou d'un de ses sels pharmaceutiquement acceptables ; et
1 à 8 % en poids de pyrrolidonecarboxylate de sodium ; dans laquelle l'acide lactique et le prednicarbate ont une pureté d'au moins 90 % et une concentration en produit(s) de dégradation inférieure à 10 % de la concentration de départ dudit acide lactique et dudit prednicarbate, pendant la durée de conservation de la composition ; et
ladite composition étant une lotion qui est une émulsion comportant une phase huileuse et une phase aqueuse, ladite composition ayant un pH de 3,0 à 6,0.

2. Composition pharmaceutique suivant la revendication 1, comprenant à 3 à 7 % en poids dudit acide lactique, 0,15 à 0,5 % en poids dudit prednicarbate et 3 à 7 % en poids dudit pyrrolidonecarboxylate de sodium.

3. Composition pharmaceutique suivant la revendication 1 ou 2, ladite composition ayant un pH de 4,0 à 5,0.

4. Composition pharmaceutique suivant l'une quelconque des revendications 1 à 3, dans laquelle ladite phase huileuse comprend une matière huileuse choisie entre une huile minérale, la vaseline, des dérivés de pétrole, des acides gras, des dérivés d'acides gras, des alcools gras, des dérivés d'alcools gras, des paraffines et leurs mélanges.

5. Composition pharmaceutique suivant l'une quelconque des revendications 1 à 4, dans laquelle ladite émulsion est formée en utilisant un émulsionnant choisi entre des esters d'acides gras de polyoxyéthylène-sorbitanne, des esters d'acides gras de sorbitanne, le stéarate de propylèneglycol, le monostéarate de glycéryle, le polyéthylèneglycol, des alcools gras, des polymères séquencés oxyde d'éthylène-oxyde de propylène, leurs dérivés, leurs sels pharmaceutiquement acceptables et leurs mélanges.

6. Composition pharmaceutique suivant la revendication 5, dans laquelle ledit émulsionnant est une association d'alcool stéarylique et d'éther cétostéarylique de polyoxy-éthylène(20), de stéarate de glycéryle et de polyéthylène-glycol-100/stéarate de glycéryle.

7. Composition pharmaceutique suivant l'une quelconque des revendications 1 à 6, dans laquelle ledit acide lactique et ledit pyrrolidonecarboxylate de sodium sont présents dans ladite phase aqueuse.

8. Composition pharmaceutique suivant l'une quelconque des revendications 1 à 7, dans laquelle ledit prednicarbate est dispersé dans l'émulsion.

9. Composition pharmaceutique suivant l'une quelconque des revendications 1 à 8, ladite composition comprenant 50 à 98 % en poids d'eau.

10. Composition pharmaceutique suivant l'une quelconque des revendications 1 à 9, ladite composition comprenant en outre un excipient choisi entre des antioxydants, des chélates, des conservateurs, des agents émollients, des agents humidifiants, des alcools alkyliques fluides, des agents épaississants, des modificateurs de pH et leurs mélanges.

11. Composition pharmaceutique suivant la revendication 10, ladite composition comprenant un modificateur de pH.

12. Composition pharmaceutique suivant la revendication 11, dans laquelle ledit modificateur de pH est l'hydroxyde de sodium.

13. Composition pharmaceutique suivant l'une quelconque des revendications 1 à 12, pour une utilisation dans le traitement d'une dermatose apte à répondre aux stéroïdes chez un mammifère par administration topique.

14. Composition pour une utilisation suivant la revendication 13, ladite dermatose apte à répondre aux stéroïdes étant choisie entre la dermatite de contact, l'eczéma, la dermatite atopique, l'ichtyose, le psoriasis, la xérodermie, la dermatite séborrhéique, la dermatite nummulaire, la dermatite de stase, la névrodermite circonscrite, des dermatophytides, la candidose, la gale, le pityriasis rosé, le lichen plan, le pityriasis rubra pilaire, la pemphigoïde bulleuse, la miliaire, l'eczéma aigu et l'eczéma chronique, le lupus érythémateux, des réactions photoallergiques, le prurit et leurs associations.
